# EUROPÄISCHE PATENTSCHRIFT

(11) **EP 3 442 450 B1**
(45) Veröffentlichungstag und Bekanntmachung des Hinweises auf die Patenterteilung: **09.08.2023**
(21) Anmeldenummer: 17715472.1
(22) Anmeldetag: 04.04.2017
(51) Int. Cl.: A61B 17/70

(54) **INSTRUMENT ZUM FÜHREN EINES STABS IN EINE IMPLANTATAUFNAHME**
INSTRUMENT FOR GUIDING A ROD INTO AN IMPLANT RECEIVING AREA
INSTRUMENT POUR GUIDER UNE TIGE DANS UN LOGEMENT D'IMPLANT

(30) Priorität: 11.04.2016 DE 102016106608
(43) Veröffentlichungstag der Anmeldung: 20.02.2019
(73) Patentinhaber: Aesculap AG, 78532 Tuttlingen (DE)
(72) Erfinder: FISCHER, Kay, 78532 Tuttlingen (DE)
(74) Vertreter: Winter, Brandl - Partnerschaft mbB
(86) Internationale Anmeldenummer: PCT/EP2017/057965
(87) Internationale Veröffentlichungsnummer: WO 2017/178283

(56) Entgegenhaltungen:
- EP-A2- 2 878 276
- WO-A2-2013/009493
- US-A1- 2008 319 477
- US-A1- 2012 191 144
- US-A1- 2016 030 093

## Beschreibung

Die vorliegende Erfindung betrifft Instrument zum Führen eines Stabs in eine Aufnahme eines Implantat, insbesondere einer Pedikelschraube, umfassend eine Kopplungseinheit zum Koppeln des Instruments mit dem Implantat, insbesondere mit einem Kopf der Pedikelschraube, eine Stabdrückereinheit aufweisend eine mit einem Außengewinde versehene und zur Kopplungseinheit in axialer Richtung positionierbare Gewindestange sowie einen mit dieser drehbar und axialfest gekoppelten oder verbundenen Stabdrücker und zumindest ein zur Kopplungseinheit axialfest angeordnetes Innengewindesegment, wobei das Innengewindesegment mit dem Außengewinde der Gewindestange in Eingriff bzw. außer Eingriff bringbar ist.

Derartige Instrumente werden zum Beispiel bei offenen Wirbelsäulen-Operationen eingesetzt, um mittels eines Stabs eine feste oder rigide Verbindung zwischen in verschiedene Wirbel eingeschraubte Pedikelschrauben zu erstellen. Das Instrument dient dazu, Stäbe, über die benachbarte Pedikelschrauben zueinander fixiert werden, in deren jeweilige Tulpe einzuführen und in dieser Position zu halten, damit der Stab dort mit einer Set-Screw gesichert werden kann. In Fällen, bei denen der Stab weit über der Tulpe platziert und von dieser beabstandet ist, z.B. bedingt durch eine Spondylolisthese (Wirbelgleiten), sind unter Umständen große Kräfte erforderlich, um den Stab in die Tulpe zu zwingen bzw. einen abgeglittenen Wirbelkörper in die gewünschte Position zu ziehen. Hierfür sind Instrumente bekannt, bei denen ein Positionieren des Stabs in Richtung eines Pedikelschraubenkopfs ausschließlich durch eine Schraubbetätigung des Instruments erfolgt. Ein Teil des Instruments greift den Kopf einer Pedikelschraube und ein anderer Teil des Instruments stützt sich auf einen in den Kopf einzudrückenden Stab ab. Die beiden Teile sind über einen Gewindemechanismus miteinander gekoppelt und können durch gegenseitiges Verschrauben einander angenähert werden, so dass der Stab in die Tulpe des Pedikelschraubenkopfs gedrückt wird bzw. die Schraube zum Stab hin gezogen wird. Nachteilig bei solchen Instrumenten ist, dass eine aufwendige und mühevolle Verstellung infolge reiner Schraubbetätigung über weite Zustellstrecken erforderlich ist. Die führt insbesondere dazu, dass das Instrument zu Reinigungszwecken nur äußerst aufwändig zu demontieren ist, da seine über den Gewindemechanismus miteinander gekoppelten Teile stets vollständig zu verschrauben bzw. zu entschrauben sind.

Ein bekanntes Instrument mit reiner Schraubbetätigung besitzt eine Implantataufnahme, eine Gewindehülse und einen vorderen Handgriff. Implantataufnahme, Gewindehülse und Handgriff sind dreh- und axialfest zueinander angeordnet oder ausgebildet. Das Instrument weist des Weiteren einen Stabdrücker und eine Gewindestange auf, die ebenfalls axialfest aber zueinander drehbar miteinander gekoppelt sind. Beide Bauteile sind im Instrument relativ zur Implantataufnahme verschiebbar gelagert. Die Gewindestange ist an ihrem proximalen, vom Stabdrücker abgewandten Ende mit einem Handgriff versehen und steht mit der Gewindehülse in Eingriff. Durch eine Drehbewegung des Handgriffs kann sie in die Gewindehülse hinein- und aus dieser herausgeschraubt werden. Eine dabei ausgeführte Translation wird infolge der vorstehend beschriebenen Kopplung mit dem Stabdrücker auf diesen übertragen, wobei der Stabdrücker nicht rotiert. Im Resultat erfolgt durch Ein- und Ausschrauben der Gewindestange in bzw. aus der Gewindehülse eine Axialpositionierung von Implantataufnahme und Stabdrücker relativ zueinander. Eine Demontage zu Reinigungszwecken ist aufwändig.

Da die Distanz, über die mittels des Instruments ein Stab in eine Pedikelschraubentulpe gedrückt werden muss, üblicherweise größer ist als 25mm, z.B. 45mm, wurden Instrumente entwickelt, die eine Schnellverstellung aufweisen. Ein solches Instrument ist zum Beispiel aus der US 2015/0100097 A1 oder der US 2015/0100098 A1 bekannt und weist einen Rastmechanismus auf. Eine Gewindestange greift in eine in radialer Richtung relativpositionierbare und gefederte Gewindeschale bzw. einen Gewindeabschnitt ein. Bei axialem Druck auf die Gewindestange verschiebt sich die Gewindeschale / der Gewindeabschnitt gegen die Federvorspannung in radiale Richtung (federt ein), so dass die Gewindestange ohne Schraubbewegung in axialer Richtung vorgeschoben werden kann, bis ein durch den Stab ausgeübter Gegendruck zu groß wird. Ab diesem Punkt kann eine weitere Axialpositioniert durch Schrauben erfolgen, wobei hohe Kräfte auf den Stab übertragen werden können. Sobald der Stab die gewünschte Endposition erreicht hat, wird eine Set-Screw in die Tulpe geschraubt und der Stab derart fixiert. Durch Druck auf einen Entriegelungsknopf, der mit der Gewindeschale bzw. dem Gewindeabschnitt wirkverbunden ist, werden die Gewinde voneinander gelöst und außer Eingriff gebracht. Die Gewindestange wird so freigeben und kann zurückgezogen werden. Vorteile dieses Mechanismus sind ein weitgehend ermüdungsfreies Arbeiten und Zeitersparnis, da nicht die gesamte Axialpositionierung durch Schrauben erfolgen muss. Da diese Instrumente aber in der Lage sind, Druckkräfte von mehreren 1000N auszuüben, stellt ein Entkoppeln von Gewindestange und Gewindeschale unter Druck in nachteiliger Weise eine Gefahr dar, umliegende Strukturen oder OP-Personal zu verletzen. Das Instrument ist zwar infolge seines Rastmechanismus schnell zu demontieren, weist aber den Nachteil auf, dass es bei einer Entkopplung zu einer unbeabsichtigten vollständigen Demontage des Instruments kommen kann, was im Rahmen einer OP, bei der mehrere Knochenschrauben zu verspannen sind, unerwünscht ist.

Die US 2008/319477 A1 offenbart ein spinales Implantationssystem, bei der eine Außengewindestange mit einem Kopplungsabschnitt zur Kopplung mit einer Aufnahmehülse einer polyaxialen Pedikelschraube axialbeweglich in einer äußeren Einheit eingefasst ist. Das Außengewinde der Gewindestange greift dabei in ein Innengewinde einer Aktuierungseinheit ein. Diese Aktuierungseinheit ist drehbar und axialfest an der äußeren Einheit befestigt. Durch Drehung der Aktuierungseinheit lässt sich die Kopplungsabschnitt in axialer Richtung bewegen.

Die EP 2 878 276 A2 offenbart ein Pedikelschrauben-Verriegelungssystem mit einem Reduzierrohr. Dieses Reduzierrohr beinhaltet eine Innenwelle, ein Griffpaar, eine Reduzierhülse und ein drehbares Rohr. Die Innenwelle weist dabei ein Gewindeende und Reduzierarme auf, die sich distal von dem Gewindeende aus erstrecken. Jeder Griff des Griffpaares hat einen Finger, der angepasst ist, um in die Aufnahmehülse einer Pedikelschraube einzugreifen. Die Reduzierhülse und das drehbare Rohr sind koaxial zur Innenwelle ausgerichtet. Das drehbare Rohr ist dabei mit der Reduzierhülse gekoppelt. Das drehbare Rohr bewegt die Reduzierhülse in Bezug auf die Reduzierarme. Der Verriegelungstreiber beinhaltet einen Gewindeabschnitt und eine stumpfe Spitze. Der Gewindeabschnitt ist konfiguriert, um in ein Gewinde in der Aufnahme der Pedikelschraube eingedreht zu werden, bis die stumpfe Spitze die Aufnahme der Pedikelschraube verriegelt. Die Reduktionsarme können einen Reduktionsschlitz zwischen sich definieren. Ein Spinalstab ist dann durch den zwischen den Reduktionsarmen definierten Reduktionsschlitz einführbar.

Ein Problem, dass viele bekannte Instrumente aufweisen, ist ihre Reinigungseignung. In der Regel sind die Instrumente möglichst schlank ausgeführt, um Hautinzisionen klein halten zu können. Dem entsprechend sind die einzelnen Bauelemente des Instruments eng miteinander platziert und schlecht zugänglich, was zu einer schlechten Reinigbarkeit führt.

Vor diesem Hintergrund liegt der vorliegenden Erfindung die Aufgabe zugrunde, ein Instrument zum Relativpositionieren, Führen und Einführen eines Stabs in eine Aufnahme eines Implantats, insbesondere einer Pedikelschraube bereitzustellen, mit dem hohe Druckkräfte zwischen Stab und Implantat übertragen werden können, und das auch für große Anfangsabstände von Stab und Implantat geeignet ist. Das Instrument soll zu Reinigungszwecken einfach zu demontieren sein, eine unbeabsichtigte Demontage soll aber stets sicher vermieden werden. Eine Gefährdung und Verletzung von an das Instrument angrenzenden Strukturen sowie von OP-Personal soll ausgeschlossen oder zumindest minimiert sein.

Diese Aufgabe wird nach der vorliegenden Erfindung gelöst durch ein Instrument nach dem Oberbegriff von Anspruch 1, das dadurch gekennzeichnet ist, dass die Stabdrückereinheit einen Anschlag aufweist, der in radialer Richtung nach außen vorspringt, und das Instrument ein zur Kopplungseinheit axialfest angeordnetes Riegelelement zum Zusammenwirken mit dem Anschlag aufweist, das gegenüber der Gewindestange in radialer Richtung positionierbar ist und durch ein eine Radialkraft aufbringendes erstes Vorspannelement in Richtung der Gewindestange in einen Eingriff mit dem Anschlag vorgespannt ist. In der nachfolgenden Beschreibung wird das das Riegelelement in Richtung der

Gewindestange vorspannende Vorspannelement als "erstes Vorspannelement" bezeichnet, um es gegenüber ggf. weiteren Vorspannelementen von Ausführungsformen des Instruments unterscheiden zu können. Die Bezeichnung "erstes Vorspannelement" beinhaltet aber nicht, dass das Instrument zwingend ein zweites Vorspannelement oder weitere Vorspannelemente aufweist.

Die Längsachsen der Gewindestange und des Stabdrückers überdecken einander, anders ausgedrückt sind sie in axialer Richtung hintereinander angeordnet. Der Anschlag, der nach der Erfindung entweder an oder in der Gewindestange und/oder an oder in dem Stabdrücker ausgebildet sein kann, springt in radialer Richtung nach außen vorspringen. Er ist nach einer Ausführungsform der Erfindung in Form eines radialen Versatz, einer Schulter oder einer Stufe ausgebildet. Es ist vorteilhaft, wenn er vollumfänglich umlaufend ausgebildet ist, da so ein unbeabsichtigtes Lösen der Gewindestange unabhängig von deren Drehpositionierung verhindert ist. Der Anschlag ist vorzugsweise auf der distalen Seite des Außengewindes ausgebildet.

Bei der Beschreibung der Erfindung verwendete Richtungsangaben, wie axial, radial und tangential, sind auf die Gewindestange und deren Orientierung bezogen. Die Bezeichnungen "distal" und "proximal" sind aus der Sicht des Operateurs auf das Instrument bezogen. In diesem Sinne bedeutet proximal "nahe dem Operateur" bzw. "zum Operateur weisend", während distal "vom Operateur entfernt" bzw. "vom Operateur wegweisend" bedeutet. Bei bestimmungsgemäßer Nutzung des Instruments ist dessen Längsachse, d.h. auch die Längsachse der Gewindestange, im Wesentlichen in Richtung der Längsachse einer Pedikelschraube ausgerichtet. Man kann daher sagen, dass das Instrument wie auch die Gewindestange jeweils ein distales und ein proximales Ende aufweisen.

Durch die Erfindung wird der Vorteil erzielt, dass zum einen durch den Gewindeeingriff zwischen der Gewindestange und dem Innengewindesegment bei feiner Verstellung und ggf. Selbsthemmung der Gewinde hohe Anpresskräfte auf einen in eine Tulpe einzupressenden Stab ausgeübt werden können. Zum anderen weist das Instrument eine Art Schnellverstellung auf, die durch Entkoppeln des Gewindeeingriffs zwischen der Gewindestange und dem Innengewindesegment bewirkt wird. Das Instrument ist daher nicht mühsam über den gesamten Verstellbereich durch relatives Verschrauben zu verstellen, sondern kann durch Entkoppeln rasch, einfach und unaufwändig zugestellt und geöffnet werden. Ein besonderer Vorteil besteht darin, dass anders als bekannten Instrumenten trotz der Schnellverstellung durch Entkoppeln von Gewindestange und Innengewindesegment ein vollständiges Lösen dieser beiden Bestandteile sicher und einfach verhindert wird, indem sichergestellt ist, dass das Riegelelement infolge seiner radialen Vorspannung mit dem Anschlag in Eingriff ist oder gelangt. Auf diese Weise wird eine Art Sicherheitsverriegelung ausgebildet. Eine vollständige Demontage ist dennoch durch nutzerseitiges Lösen des Riegelelements aus dem Eingriff mit dem Anschlag einfach und schnell möglich. Dabei ist sichergestellt, dass die vollständige Demontage nicht versehentlich, sondern stets nur beabsichtigt erfolgt.

Bei einem Einsatz des erfindungsgemäßen Instruments kann die Gewindestange durch Entkoppeln vom Innengewindesegment in axiale Richtung schnellpositioniert werden. Die Gewindestange kann so ohne Schraubbewegung in axialer Richtung vorgeschoben werden, bis ein durch den Stab ausgeübter Gegendruck zu groß wird. Ab diesem Punkt kann eine weitere Axialpositionierung durch Schrauben erfolgen, wobei hohe Kräfte auf den Stab übertragen werden können. Sobald der Stab die gewünschte Endposition erreicht hat, kann dieser mittels einer Set-Screw oder Ähnlichem am Implantat gesichert werden. Zum Entfernen des Instruments kann die Gewindestange zunächst durch Herausschrauben aus dem Innengewindesegment soweit gelöst werden, bis sie druckentlastet ist. Durch Entkoppeln des Außengewindes der Gewindestange vom Innengewinde des Innengewindesegments kann dann die Gewindestange mittels einer reinen Axialpositionierung zurückgezogen werden. Nach der Erfindung ist diese frei axiale Positionierbarkeit jedoch durch den Anschlag der Gewindestange und das mit diesem in Eingriff stehende oder in Eingriff gelangende Riegelelement begrenzt. Es ist ein besonderer Vorteil der Erfindung, dass neben einem weitgehend ermüdungsfreiem Arbeiten und einer Zeitersparnis, da nicht die gesamte Axialpositionierung durch Schrauben erfolgen muss, ein unbeabsichtigtes vollständiges Lösen oder Entfernen der Gewindestange aus dem Instrument nicht möglich ist.

Vorteilhafte Ausführungsformen der Erfindung sind in den Unteransprüchen beansprucht und werden nachfolgend näher erläutert.

Eine Ausführungsform der Erfindung ist dadurch gekennzeichnet, dass der Außendurchmesser (maximale Durchmesser) des Anschlags kleiner ist als der Fußdurchmesser des Außengewindes. Auf diese Weise ist sichergestellt, dass ein Eingriff zwischen dem Innengewindesegment und dem Anschlag nicht möglich ist. So kann eine Beschädigung des Innengewindes sicher vermieden werden. Des Weiteren kann vermieden werden, dass das Innengewindesegment bei einem vollständigen Entfernen der Gewindestange aus dem Instrument mit dem Anschlag in Eingriff gelangt und so eine Demontage erschwert.

Es ist von besonderem Vorteil, wenn in der Gewindestange ein axialer gewindefreier Abschnitt ausgebildet ist, dessen Außendurchmesser kleiner ist als der Fußdurchmesser des Außengewindes. Die axiale Länge eines derartigen Abschnitts kann nach einer Ausführungsform größer als die axiale Länge des Innengewindes des Innengewindesegments sein. Durch den gewindefreien Abschnitt wird eine Art Leerlaufbereich erzeugt, in dem die Gewindestange gegenüber dem Innengewindesegment verdreht werden kann, ohne dass es zu einem Vorschub in axialer Richtung kommt. Auf diese Weise kann das Instrument besonders einfach an einer Pedikelschraube angesetzt und zum Einpressen des Stabs vorbereitet werden. Der gewindefreie Abschnitt kann insbesondere auf der distalen Seite des Außengewindes ausgebildet sein. Eine weitere Ausführungsform der Erfindung sieht vor, dass sowohl distal als auch proximal ein jeweils ein gewindefreier Abschnitt ausgebildet ist.

Eine Ausführungsform der Erfindung ist dadurch gekennzeichnet, dass das Instrument ein Entriegelungselement aufweist, das mit dem Riegelelement zusammenwirkt. Das Riegelelement kann insbesondere durch eine nutzerseitige Betätigung des Entriegelungselements, unter Umständen gegen die durch das erste Vorspannelement aufgebrachte Vorspannung, in radialer Richtung nach außen bewegt werden und derart vom Anschlag entkoppelbar sein. Vorzugsweis ist das Entriegelungselement in radialer Richtung relativ zur Gewindestange positionierbar.

Nach einer weiteren Ausführungsform kann das Entriegelungselement in einem Gehäuse aufgenommen sein. Darin kann es insbesondere mittels einer Feder als erstes Vorspannelement in Richtung von der Gewindestange fort, also nach radial außen, vorgespannt sein. In einer besonderen Ausführungsform ist diese Feder zwischen Riegelelement und Entriegelungselement angeordnet und verspannt diese beiden Element gegeneinander. Eine Vorspannung des Riegelelements in Richtung der Gewindestange kann dabei bewirkt werden, indem das Entriegelungselement mit einem im Gehäuse gebildeten Anschlag zusammenwirken kann, der eine Begrenzung für eine radial von der Gewindestange fort gerichtete Bewegung des Entriegelungselements bildet. Auf diese Weise bildet das Entriegelungselement eine Art schwimmende Lagerung oder Aufnahme für das Riegelelement aus.

Es ist von besonderem Vorteil, wenn das Riegelelement eine Kontaktfläche für die Gewindestange aufweist, mit der es am Kopfdurchmesser (Außendurchmesser) des Außengewindes anliegend in axialer Richtung auf der Gewindestange gleiten kann, ohne mit dem Außengewinde in Eingriff zu gelangen. Auf diese Weise wird eine funktionale Trennung der Sicherheitsverriegelung von der Gewindeverstellung erreicht, was eine besonders einfache Bedienung des Instruments sicherstellt. Des Weiteren kann das Riegelelement eine nach Art einer schiefen Ebene angeordnete, also schräg zur Längsachse der Gewindestange ausgerichtete, Einlauffläche zum Kontakt mit dem distalen Ende der Gewindestange aufweisen, die ein Einschieben der Gewindestange in das Instrument erleichtert.

Eine weitere Ausführungsform der Erfindung ist dadurch gekennzeichnet, dass das Innengewindesegment gegenüber der Gewindestange in radialer Richtung positionierbar ist und durch ein eine Radialkraft aufbringendes zweites Vorspannelement in Richtung der Gewindestange vorgespannt ist, wobei das Innengewindesegment vorzugsweise in einem Gehäuse aufgenommen ist und vorzugsweise mittels einer Feder als zweites Vorspannelement in Richtung der Gewindestange vorgespannt ist. Das Innengewindesegment ist insbesondere durch Radialpositionierung mit dem Außengewinde der Gewindestange in Eingriff bzw. außer Eingriff bringbar. Bei in radialer Richtung zur Gewindestange positioniertem Innengewindesegment, im Folgenden auch erste Position bezeichnet, stehen das Außengewinde der Gewindestange und das Innengewinde des Innengewindesegments miteinander in Eingriff. Bei in radialer Richtung von der Gewindestange fort positioniertem Innengewindesegment, im Folgenden auch als zweite Position bezeichnet, sind sie außer Eingriff gebracht. In der zweiten Position ist daher die Gewindestange relativ zum Innengewindesegment frei axialpositionierbar, das heißt axial sowohl in distaler als auch in proximaler Richtung positionierbar, insbesondere durch nutzerseitige Einwirkung verschiebbar.

Das Innengewindesegment kann insbesondere in Form einer Gewindeschale mit einem zur Gewindestange weisenden Innengewinde oder Innengewindeabschnitt ausgebildet sein. Im Rahmen der Erfindung weist das Instrument zumindest ein Innengewindesegment auf. Es ist außerdem im Rahmen, wenn das Instrument zwei, drei oder vier Innengewindesegmente in umfänglicher Richtung voneinander beabstandete Innengewindesegmente aufweist, so dass ein gleichmäßiger Gewindeeingriff sichergestellt ist.

Das Innengewindesegment kann insbesondere in dem Gehäuse aufgenommen sein, in dem auch das Riegelelement aufgenommen ist sowie das Entriegelungselement gelagert ist. Es kann insbesondere mittels einer Feder in Richtung der Gewindestange vorgespannt sein, welche Feder am oder im Gehäuse geführt und in radialer Richtung nach außen abgestützt ist.

Eine Ausführungsform der Erfindung ist dadurch gekennzeichnet, dass Gewindeflanken der Gewindestange und Gewindeflanken des Innengewindesegments, die einander zugewandt sind, jeweils einen Hinterschnitt aufweisend ausgebildet sind. Unter einem Hinterschnitt einer Gewindeflanke im Sinne der Erfindung ist eine Ausbildung einer Gewindeflanke zu verstehen, bei der eine Gewindeflanke einen in radialer Richtung äußeren Bereich aufweist, der gegenüber einem in radialer inneren Bereich dergleichen Gewindeflanke in axialer Richtung vorsteht. Dies kann zum Beispiel der Fall sein, wenn in eine Gewindeflanke eine Hintergreifung oder Nut eingebracht ist, oder der Neigungswinkel a einer Flanke negativ ist (bei einem üblichen metrischen Gewinde ist der Neigungswinkel jeder Flanke positiv).

Infolge des Hinterschnitts sind das Außengewinde der Gewindestange und das Innengewinde des Innengewindesegments bei Eingriff ineinander miteinander verhakt. Bei einem auf die Gewindestange ausgeübten Druck, der so in axialer Richtung wirkt, dass die mit Hinterschnitt versehenen Gewindeflanken von Gewindestange und Innengewindesegment gegeneinander gedrückt werden, ist eine Verlagerung des Innengewindesegments infolgedessen in radialer Richtung nicht möglich. Das Gewinde ist quasi gesichert und gehemmt.

Die jeweils den mit Hinterschnitt versehenen Gewindeflanken der Gewindestange gegenüberliegenden "normalen" Gewindeflanken der Gewindestange und die jeweils den mit Hinterschnitt versehenen Gewindeflanken des Innengewindesegments gegenüberliegenden "normalen" Gewindeflanken des Innengewindesegments können im Sinne der Erfindung bei einem auf die Gewindestange wirkenden Axialdruck entsprechender Höhe wie eine schiefe Ebene oder ein Umlenkgetriebe wirken. Ist ein Axialdruck, der so in axialer Richtung wirkt, dass die ohne Hinterschnitt ausgebildeten Gewindeflanken von Gewindestange und Innengewindesegment gegeneinander gedrückt werden, ausreichend hoch, wird infolge der schräg zur Axialrichtung ausgerichteten Gewindeflanken das Innengewindesegment in radiale Richtung nach außen verlagert oder positioniert. Das Außengewinde der Gewindestange und das Innengewinde des Innengewindesegments gelangen so außer Eingriff und die Gewindestange kann in axialer Richtung durchrutschen. Auf diese Weise ist eine Schnellpositionierung der Gewindestange in axialer Richtung möglich. Insgesamt ist bei dieser Ausführungsform eine Axialverschiebung in die eine Richtung möglich, während sie in die andere entgegen gerichtete axiale Richtung durch den Hinterschnitt gehemmt ist und die Gewindestange in diese Richtung nur durch Schrauben bewegt werden kann.

Bei einer Ausführungsform kann eine mit Hinterschnitt ausgebildete Gewindeflanke einen gegenüber einer Normalen zur jeweiligen Gewindeachse geneigten negativen Flankenwinkel α aufweisen, der vorzugsweise in einem Bereich von ca. -10° bis ca. -1°, bevorzugter von ca. -8° bis ca. -2°, bevorzugter von ca. -6° bis ca. -3° noch bevorzugter von ca. -5° bis ca. -4° liegt. Es liegt auf der Hand, dass die Flankenwinkel der Gewindestange auf die des Innengewindesegments abgestimmt sind.

Von besonderem Vorteil ist, wenn die mit Hinterschnitt ausgebildeten Flankenwinkel der Gewindestange proximalseitig und die mit einem Hinterschnitt ausgebildeten Flankenwinkel der Gewindehülse distalseitig angeordnet sind. In diesem Fall ist ein Positionieren der Gewindestange in proximale axiale Richtung bei in Eingriff stehenden Gewinden von Gewindestange und Innengewindesegment nicht möglich. Ein Lösen des Gewindeeingriffs durch eine Verlagerung des Innengewindesegments in radialer Richtung nach außen ist bei unter einer Druckbelastung stehenden Gewindestange nicht möglich, da der Hinterschnitt eine solche Bewegung sperrt. Die in Eingriff stehenden Gewinde sind gesichert. Wird jedoch die Gewindestange durch teilweises Lösen und Herausschrauben druckentlastet, kann der Gewindeeingriff trotz Hinterschnitt gelöst werden, wobei es zu einer geringen Verlagerung der Gewindestange in die distale axiale Richtung kommt.

Bei einer Weiterbildung dieser Ausführungsform sind die einer Flanke mit Hinterschnitt jeweils gegenüberliegenden Gewindeflanken mit einem positiven Flankenwinkel β ausgebildet. Auf diese Weise ist ein Positionieren der Gewindestange in distaler axialer Richtung auch bei in Eingriff stehenden Gewinden möglich, indem auf die Gewindestange ein ausreichend hoher axialer Druck aufgebracht wird, bei dem das Innengewindesegment über den positiven Flankenwinkel der distalseitigen Flanken der Gewindestange sowie der proximalseitigen Flanken des Innengewindesegments, die nach Art einer schiefen Ebene wirken, in radialer Richtung gegen die auf es wirkende Vorspannung nach außen gedrängt wird.

Nach einer Ausführungsform der Erfindung sind das Riegelelement und das Innengewindesegment auf einander diametral gegenüberliegenden Seiten der Gewindestange angeordnet. Dabei kann das Entriegelungselement durch nutzerseitige Betätigung in eine erste Richtung, insbesondere in radialer Richtung auf die Gewindestange zu, sowie in eine zweite Richtung, insbesondere in radialer Richtung von der Gewindestange fort, positionierbar sein. Auf diese Weise kann mit einem einzigen Entriegelungselement als nutzerseitige Betätigungseinheit entweder eine Entriegelung des Innengewindes des Innengewindesegments vom Außengewinde der Gewindestange oder eine Entriegelung des Riegelelements vom Anschlag bewirkt werden. Dies ist besonders nutzerfreundlich. Insbesondere kann das Entriegelungselement bei Positionierung in die erste Richtung das Innengewindesegment gegen die Radialkraft des Vorspannelements von der Gewindestange fortbewegt werden und mit deren Außengewinde außer Eingriff gebracht werden, und bei Positionierung in die zweite Richtung das Riegelelement gegen die Radialkraft des Vorspannelements von der Gewindestange fort bewegt und mit deren Anschlag außer Eingriff gebracht werden.

Die Kopplungseinheit ist dazu eingerichtet, eine Kopplung des Instruments mit dem Implantat zu bewirken. Bei dem Implantat handelt es sich vorzugsweise um eine Pedikelschraube, die in einen Wirbel einer Wirbelsäule eingeschraubt ist. Vorzugsweise wird die Kopplungseinheit mit einem Pedikelschraubenkopf gekoppelt. Zum Koppeln des Instruments mit dem Implantat kann die Kopplungseinheit eine Implantataufnahme ausbilden oder aufweisen. Eine solche Implantataufnahme kann insbesondere zwei oder drei einander gegenüberliegende Koppelarme umfassen. Diese sind an ihrem jeweiligen distalen Ende mit einer Koppelstruktur versehen. Zumindest die distalen Enden der Koppelarme sind in radiale Richtung voneinander beabstandbar oder spreizbar, können im beabstandeten Zustand an einem Implantat angeordnet und durch Schließen der distalen Enden der Koppelarme mit diesen gekoppelt und daran fest angeordnet werden.

Die Gewindestange kann zur direkten oder indirekten Anlage an einem in ein Implantat einzudrückenden Stab ausgebildet sein. Im letzten Fall kann sie mit einer Stabdrückereinheit oder einem Stabdrückerelement verbunden sein und über diese den Stab kontaktieren und positionieren/verlagern. Vorzugsweise sind die Gewindestange und der Stabdrücker zueinander um die Längsachse drehbar miteinander gekoppelt, so dass Drehungen der Gewindestange um ihre Längsachse beim Schrauben nicht auf den Stab übertragen werden.

Bei einer Ausführungsform kann die Stabdrückereinheit mit den Koppelarmen der Kopplungseinheit zusammenwirken, beispielsweise indem die Koppelarme durch Öffnungen in der Stabdrückereinheit geführt sind, so dass die Koppelarme bei einer Positionierung der Stabdrückereinheit in distale Richtung automatisch geschlossen oder in im geschlossenen Zustand am Implantat gesichert werden.

Es ist von Vorteil zum Einschrauben einer Set-Screw in das Implantat bzw. die Pedikelschraube, wenn die Gewindestange hohl mit einem in axialer Richtung durchgehenden Durchgangskanal ausgebildet ist. Der Durchgangskanal ist derart dimensioniert, dass die Set-Screw wie auch ein Instrument zum Einschrauben der Set-Screw hindurch passen.

Man kann auch sagen, dass die Erfindung ein eingangs genanntes Instrument mit einem Rastmechanismus in verbesserter Form mit weiteren Sicherheitsfeatures betrifft. Durch die Erfindung können insbesondere die folgenden Vorteile erzielt werden: es ermöglicht eine platzsparende Zug-/Druckknopfbedienung zum Entriegeln und Demontieren, es ist für Reinigung zerlegbar, es hat wenige Komponenten und dadurch ein reduziertes Gewicht und es lässt sich schließlich einfach bedienen.

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:
Fig. 1 ein Instrument nach der Erfindung in einer perspektivischen Ansicht,
Fig. 2 eine Schnittansicht eines Rastmechanismus des Instruments der Fig. 1 in Richtung der Längsachse des Instruments,
Fig. 3 eine Schnittansicht des Rastmechanismus der Fig. 2 quer zur Axialrichtung in einer ersten Funktionsstellung,
Fig. 4 eine perspektivische teilweise freigestellte Ansicht des Rastmechanismus der Fig. 2 in der ersten Funktionsstellung,
Fig. 5 eine Schnittansicht des Rastmechanismus der Fig. 2 quer zur Axialrichtung in einer zweiten Funktionsstellung,
Fig. 6 eine perspektivische teilweise freigestellte Ansicht des Rastmechanismus der Fig. 2 in der zweiten Funktionsstellung

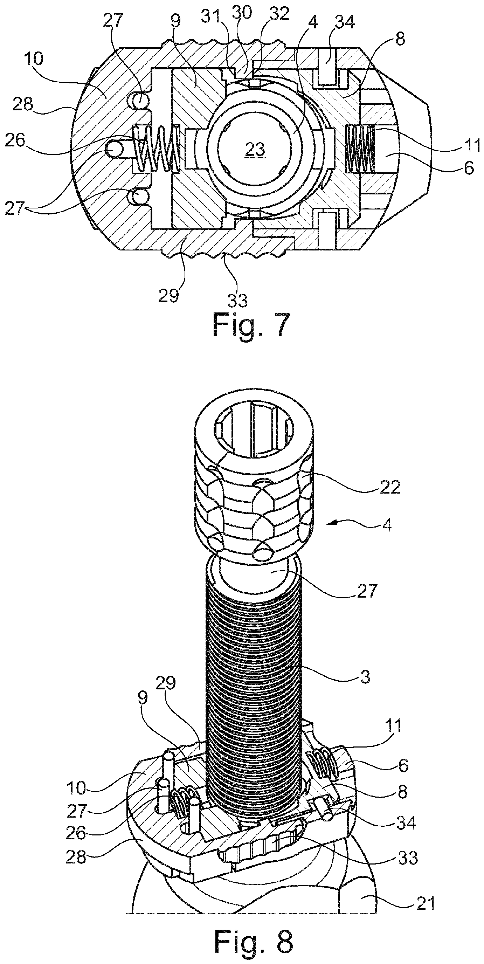

Fig. 7 eine Schnittansicht des Rastmechanismus der Fig. 2 quer zur Axialrichtung in einer dritten Funktionsstellung,
Fig. 8 eine perspektivische teilweise freigestellte Ansicht des Rastmechanismus der Fig. 2 in der dritten Funktionsstellung,
Fig. 9 eine Schnittansicht des Rastmechanismus der Fig. 2 quer zur Axialrichtung in einer vierten Funktionsstellung,

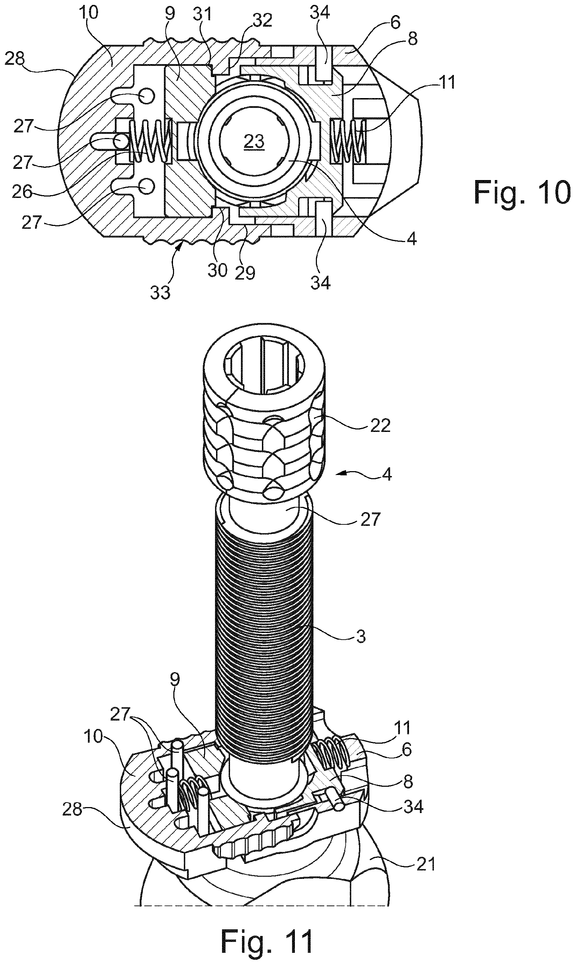

Fig. 10 eine Schnittansicht des Rastmechanismus der Fig. 2 quer zur Axialrichtung in einer fünften Funktionsstellung,
Fig. 11 eine perspektivische teilweise freigestellte Ansicht des Rastmechanismus der Fig. 2 in der fünften Funktionsstellung,
Fig. 12 eine seitliche Ansicht eines Abschnitts der Gewindestange,
Fig. 13 eine Aufsicht auf den Mechanismus des Instruments ohne Gewindestange,
Fig. 14 eine perspektivische Ansicht des Mechanismus ohne Gehäuse,
Fig. 15 die Ansicht der Fig. 14 mit teilgeschnittenem Gehäuse und
Fig. 16 einen Teilschnitt durch Innen- und Außengewinde.

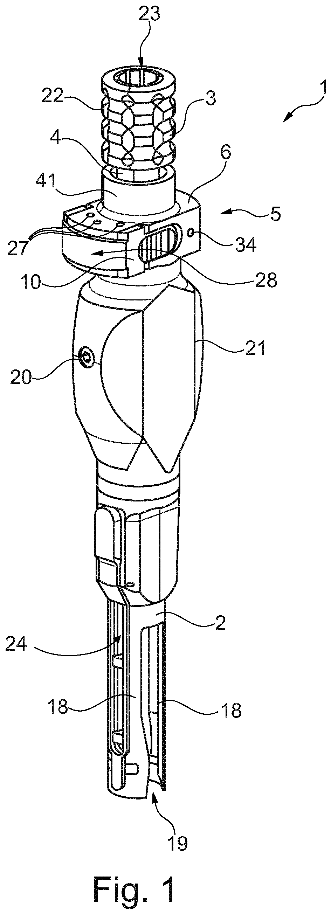

In Figur 1 ist ein erfindungsgemäßes Instrument 1 zum Relativpositionieren und/oder Führen und/oder Einführen eines in den Figuren nicht dargestellten Stabs in eine Aufnahme eines ebenfalls nicht dargestellten Implantats, wie zum Beispiel in einen Kopf einer Pedikelschraube, in einer perspektivischen Ansicht dargestellt. Das Instrument 1 umfasst im Wesentlichen eine Kopplungseinheit 2, eine mit einem Außengewinde 3 versehene und zur Kopplungseinheit 2 in axialer Richtung positionierbare Gewindestange 4 und einen zur Kopplungseinheit 2 axialfest angeordneten Rastmechanismus 5.

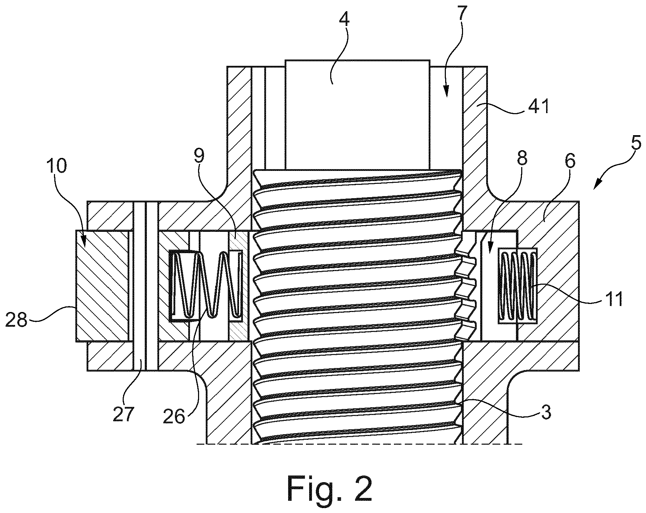

Der Rastmechanismus 5 ist zusammen mit einem Abschnitt der Gewindestange 4 vergrößert unter anderem in Figur 2 dargestellt. Er weist im Wesentlichen ein Gehäuse 6 mit einer sich in axialer Richtung erstreckenden Durchgangsöffnung 7, ein Innengewindesegment 8, ein Riegelelement 9 und ein Entriegelungselement 10 auf. Das Gehäuse 6 weist eine zylinderförmige Führung 41 auf, über die die Gewindestange 4 geführt und relativ zum Gehäuse 6 und den darin aufgenommenen und gelagerten Einheiten positioniert ist.

Das Innengewindesegment 8 ist gegenüber der Gewindestange 4 in radialer Richtung positionierbar und durch ein eine Radialkraft aufbringendes Vorspannelement 11 (zweites Vorspannelement) in Form einer Druckfeder 11 in Richtung der Gewindestange 4 vorgespannt. Infolge der radialen Positionierbarkeit kann das Innengewindesegment 8 durch Verschieben oder Platzieren in radialer Richtung mit dem Außengewinde 3 der Gewindestange 4 in Eingriff bzw. außer Eingriff gebracht werden.

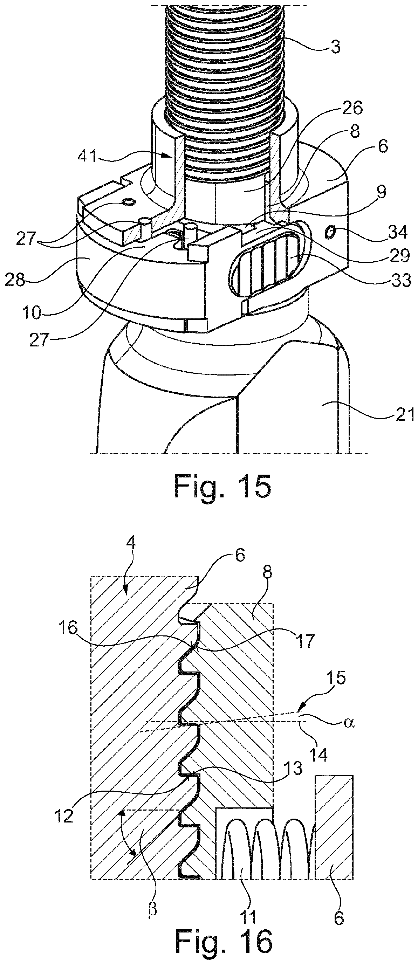

Gewindeflanken 12 der Gewindestange 4 und Gewindeflanken 13 des Innengewindesegments 8, die einander zugewandt sind, sind jeweils einen Hinterschnitt aufweisend ausgebildet. Zur Verdeutlichung des Hinterschnitts, siehe insbesondere Figur 16, ist eine Normale 14 zur Längsachse der Gewindestange 4 eingezeichnet. Ebenfalls eingezeichnet ist eine Linie 15, die sich mit den Gewindeflanken 12, 13 überdeckt. Zwischen der Normalen 14 und der Linie 15 erstreckt sich ein negativer Flankenwinkel α. Jede einer mit Hinterschnitt versehenen Gewindeflanke 12 des Außengewindes 3 der Gewindestange 4 gegenüberliegende Gewindeflanke 16 ist mit einem positiven Flankenwinkel β ausgebildet. Ebenfalls ist jede einer mit Hinterschnitt versehenen Gewindeflanke 13 des Innengewindesegments 8 gegenüberliegende Gewindeflanke 17 mit einem positiven Flankenwinkel β ausgebildet.

Figur 1 zeigt, dass die Kopplungseinheit 2 zwei einander gegenüberliegende Koppelarme 18 aufweist, die jeweils an ihrem distalen Ende mit einer Koppelstruktur 19 versehen sind, zur zumindest axialfesten Ankopplung des Instruments 1 am Kopf einer Pedikelschraube. Die Kopplungseinheit 2 ist mittels Schrauben oder Nieten 20 drehfest und axialfest mit einem distalen Griffelement 21 verbunden. Das Griffelement 21 wiederum ist drehfest und axialfest mit dem Gehäuse 6 des Rastmechanismus 5 verbunden.

Figur 2 zeigt, dass die Gewindestange 4 in der Durchgangsöffnung 7 des Gehäuses 6 angeordnet ist. Die Gewindestange 4 ist an ihrem proximalen Ende mit einem proximalen Griffelement (in den Figuren nicht dargestellt) versehen, das über eine Aufnahme 22 mit der Gewindestange 4 drehfest gekoppelt ist, und ihrerseits im Wesentlichen hohlzylinderförmig mit einem in Axialrichtung durchgehenden Durchgangskanal 23 versehen. An ihrem distalen Ende ist die Gewindestange 4 axialfest mit einem Stabdrücker 24 verbunden. Die Gewindestange 4 und der Stabdrücker 24 sind relativ zueinander um die Längsachse der Gewindestange 4 drehbar.

Insgesamt kann man sagen, dass die Kopplungseinheit 2, das distale Griffelement 21 und das Gehäuse 6 mit dem darin aufgenommenen Innengewindesegment 8, dem Riegelelement 9, das man auch als Verriegelungsfalle bezeichnen kann, und dem Entriegelungselement 10 eine erste Einheit des Instruments 1 bilden. In ähnlicher Weise bilden die Gewindestange 4, der Stabdrücker 24 und das proximale Griffelement eine zweite Einheit des Instruments 1. Die erste Einheit und die zweite Einheit des Instruments sind über einen Eingriff des Außengewindes 3 der Gewindestange 4 in das Innengewinde 25 des Innengewindesegments 8 miteinander gekoppelt.

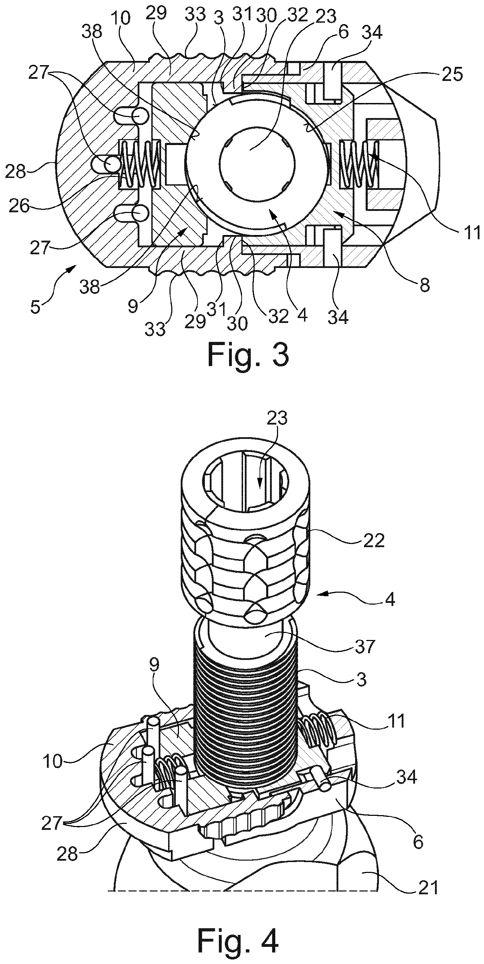

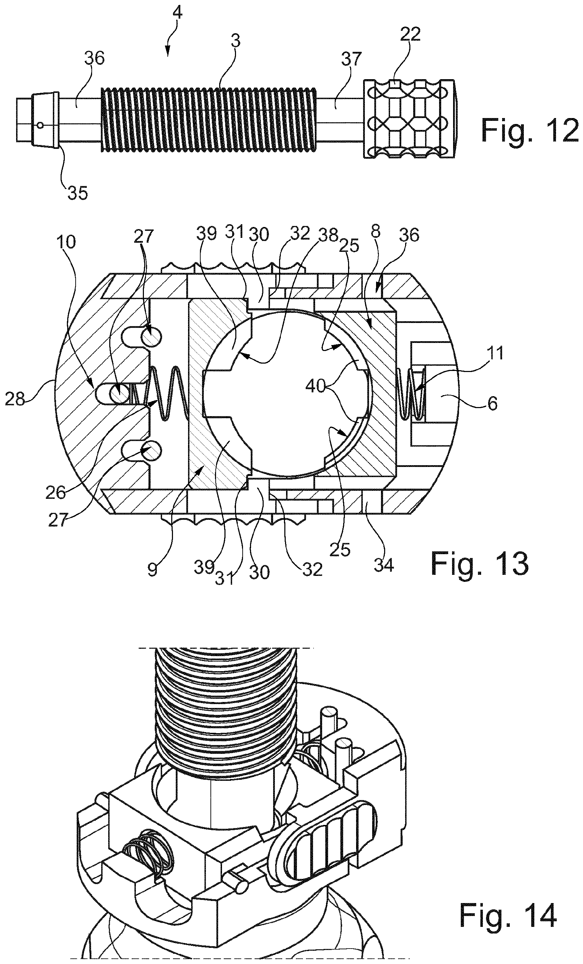

Das Innengewindesegment 8 ist mittels der Feder 11 in radialer Richtung nach innen, also in Richtung der Gewindestange 7 vorgespannt sind. Diese ist radial außen am Gehäuse 6 abgestützt. Das Riegelelement 8 ist mittels einer Druckfeder 26 (erstes Vorspannelement) in radialer Richtung gegen die Gewindestange 4 vorgespannt. Die Druckfeder 26 ist in einem Ruhezustand radial außen an einem mittleren von drei Führungstiften 27 abgestützt. Diese lagern ein Entriegelungselement 10 in radialer Richtung positionierbar im Gehäuse 6, dessen radial äußeres Ende aus dem Gehäuse 6 hinaus ragt und als Betätigungsknopf 28 ausgebildet ist. Im vorstehend genannten Ruhezustand befindet sich das Entriegelungselement 10 in radialer Richtung beabstandet von der Gewindestange 4 (Figur 3). Wird der Betätigungsknopf 28 betätigt, also radial in Richtung der Gewindestange 4 verlagert, gelangt die Druckfeder 26 mit ihm in Anlage und stützt sich daran ab. Das Entriegelungselement 10 weist, siehe insbesondere in Figur 13, zwei seitliche Arme 29 auf, die sich beiderseits des Knopfs 28 tangential zur Gewindestange 4 erstrecken. Auf ihren der Gewindestange 4 jeweils zugewandten Seiten weisen sie jeweils einen Anschlagzapfen 30 auf, mit einer ersten Anschlagfläche 31 und einer zweiten Anschlagfläche 32. Die erste Anschlagfläche 31 ist zum Zusammenwirken mit dem Riegelelement 9 und die zweite Anschlagfläche 32 zum Zusammenwirken mit dem Innengewindesegment 8 ausgebildet. Auf den äußeren Seiten der Arme 29 ist jeweils eine Griffstruktur 33 ausgebildet (siehe zum Beispiel in Figur 3). Figur 13 zeigt, dass das Innengewindesegment 8 mit Stiften 34 im Gehäuse 6 geführt ist.

Das radial innere Ende des Riegelelements 9 ist mit zwei Kontaktflächen 38 sowie axial darüber angeordneten Gleitflächen 39 versehen. Die Kontaktflächen 38 sind derart ausgebildet, dass das Riegelelement 9 auf der äußeren Mantelfläche (Außendurchmesser) des Außengewindes 3 der Gewindestange 4 aufliegen und in axialer Richtung darauf gleiten kann, ohne mit dem Außengewinde 3 in Gewindeeingriff zu gelangen. Die Gleitflächen 39 erleichtern ein axiales Einschieben der Gewindestange 4.

Da die Gewindestange 4 während einer OP in der Regel mehrmals zurückgezogen werden muss, zum Beispiel um einen Stab in mehrere Pedikelschrauben einzupressen, ist nach der Erfindung vorgesehen, dass sich das Instrument 1 dabei nicht unbeabsichtigt zerlegt. Zu diesem Zweck ist die Gewindestange 4 mit einem Anschlag 35 ausgebildet (siehe insbesondere in Figur 12). Distal des Außengewindes 3 ist ein gewindefreier Abschnitt 36 ausgebildet. Dessen Durchmesser ist kleiner als der Fußdurchmesser (Innendurchmesser) des Außengewindes 3. Der Anschlag 35 ist distal des gewindefreien Abschnitts 36 platziert. Ein zweiter gewindefreier Abschnitt 37, der im vorliegenden Beispiel ohne Funktion ist, ist proximal des Außengewindes 3 ausgebildet.

Das Instrument 1 wird wie folgt verwendet:
Zunächst wird die Gewindestange 4 in die Durchgangsöffnung 7 des Gehäuses 6 eingeschoben. Dabei gelangt ihr distales Ende mit den Gleitflächen 39 des Riegelelements 9 in Anlage, wodurch das Riegelelement 9 gegen die Spannung der Feder 11 radial nach außen gedrängt wird. Gleiches gilt für das Innengewindesegment 8, das ähnliche Gleitflächen 40 aufweist. Die Gewindestange 4 ist nun in die Durchgangsöffnung 7 des Gehäuses 6 eingeschoben und steht mit dem Innengewindesegment 8 in Gewindeeingriff.

Mit montierter Gewindestange 4 wird in einer Ausgangssituation das Instrument 1 mit der Pedikelschraube gekoppelt. Zu Beginn eines Vorgangs zum Andrücken eines Stabs an eine Pedikelschraube ist die zweite Einheit bestehend aus proximalem Griffelement 22, Gewindestange 4 und Stabdrücker 24 in proximaler Richtung aus der bereits beschriebenen ersten Einheit herausgezogen. Das Instrument 1 wird in diesem Zustand mit der Koppelstruktur 19 am Kopf der Pedikelschraube angeordnet und gekoppelt, wobei der einzudrückende Stab zwischen den beiden Koppelarmen 18 platziert ist.
(nach unten) gedrückt werden. Dabei wird das Innengewindesegment 8 durch die positiven Flankenwinkel β durch das Außengewinde 3 radial nach außen gedrückt, bis die Gewindestange 4 und das Innengewindesegment 8 außer Eingriff sind. Die Gewindestange kann axial verschoben werden, bis sie bzw. der Stabdrücker 24 an der Pedikelschraube anliegt. Infolge der radialen Vorspannung durch die Feder 11 wird der Gewindeeingriff wieder hergestellt. Eine weitere Axialpositionierung erfolgt nun durch Verdrehen der Gewindestange 4 und Herausschrauben aus dem Innengewindesegment 8.

Bei wieder ineinander greifenden Gewinden "verhaken" sich diese infolge des Hinterschnitts. Insgesamt wird durch den Hinterschnitt der Gewinde bewirkt, dass die Gewindestange 4 in eine Richtung, nämlich im vorliegenden Ausführungsbeispiel in die distale Richtung, ohne Schraubbewegung durch das Gehäuse geschoben werden kann (mit Verschieben des Innengewindesegments 8 nach radial außen), während eine reine Axialbewegung in die andere Richtung (proximal) verhindert wird. Das bewirkt den Vorteil, dass die mit dem Stabdrücker 24 verbundene Gewindestange 4 soweit von Hand in distaler Richtung eingeschoben werden kann, bis die vom Stab auf den Stabdrücker 24 ausgeübte Gegenkraft zu groß wird. Dann erfolgt eine weitere Zustellung in distaler Richtung durch Einschrauben der Gewindestange 4. Bei den vorstehend beschriebenen Vorgängen kann die Gewindestange 4 wegen des Hinterschnittgewindes und der dadurch bewirkten Selbsthemmung in jeder beliebigen Lage losgelassen werden und hält trotzdem die Position.

Da der Rastmechanismus 5 ein Hinterschnittgewinde besitzt, ist ein unbeabsichtigtes Auskoppeln des Außengewindes 3 vom Innengewinde des Innengewindesegments 8, zum Beispiel durch ein ungewolltes Ausfedern gegen die Federvorspannung, ausgeschlossen, denn eine höhere axiale Gegenkraft auf den Stab führt zu einem stärkeren Ineinandergreifen der Gewindeflanken. Des Weiteren ist infolge des Hinterschnitts das Entriegelungselemente 10 über eine Anlage von Anschlagzapfen 30 und Anschlagflächen 31 blockiert, da es darüber mit dem Innengewindesegment 8 zusammenwirkt, dessen Bewegen aufgrund des blockierten, unter Last stehenden Hinterschnittgewindes nicht möglich ist. Eine versehentliche Entriegelung ist somit nicht möglich.
Anschlagzapfen 30 und Anschlagflächen 31 blockiert, da es darüber mit dem Innengewindesegment 8 zusammenwirkt, dessen Bewegen aufgrund des blockierten, unter Last stehenden Hinterschnittgewindes nicht möglich ist. Eine versehentliche Entriegelung ist somit nicht möglich.

Hat der Stab durch Verschrauben der Gewindestange 4 gegenüber dem Innengewindesegment 8 im Kopf der Pedikelschraube seine bestimmungsgemäße Position erreicht, wird eine in den Figuren nicht gezeigte Set-Screw durch den Durchgangskanal 23 der Gewindestange 4 hindurch festgeschraubt. Sie hält den Stab in der Schraube bzw. im Kopf, so dass das Instrument 1 nun gelöst und abgekoppelt werden kann. Dazu muss zunächst die Gewindestange 4 wenige Umdrehungen gegen die Einschraubrichtung gedreht werden.

Die Gewindestange 4 ist bei einer Axialbewegung in distaler Richtung reibgehemmt, indem sie selbst und/oder der Stabdrücker 24 mit einem axialfesten Element des Instruments 1, zum Beispiel mit dem distalen Griffelement 21 in Reibanlage stehen. Die Reibhemmung ist derart bemessen, dass beim Herausschrauben der Gewindestange 4 ohne zusätzliche Druckbelastung durch den Stab aus dem Innengewindesegment 8 eine Axialverschiebung der Gewindestange 4 verhindert wird. Infolgedessen wird das Innengewindesegment 8 gegen die Vorspannung der Feder 11 in radialer Richtung nach außen deplatziert. Wenn die Gewindestange 4 ausreichend gelöst ist und wirkt auf sie kein vom Stab ausgeübter Druck mehr (was bei mittels der Set-Screw gesichertem Stab der Fall ist), schnappen die Gewindegänge des Innengewindesegments 8 nach etwa einer Umdrehung der Gewindestange 4 in den nächsten Gewindegang des Außengewindes 3 ein, wodurch ein akustisch wahrnehmbares Klicken dem Nutzer anzeigt, dass die Gewindestange 4 druckentlastet ist, und er die Gewindestange 4 durch Druckbetätigung des Entriegelungselements 10, 28 gefahrlos entriegeln und in axialer Richtung zurückziehen kann.

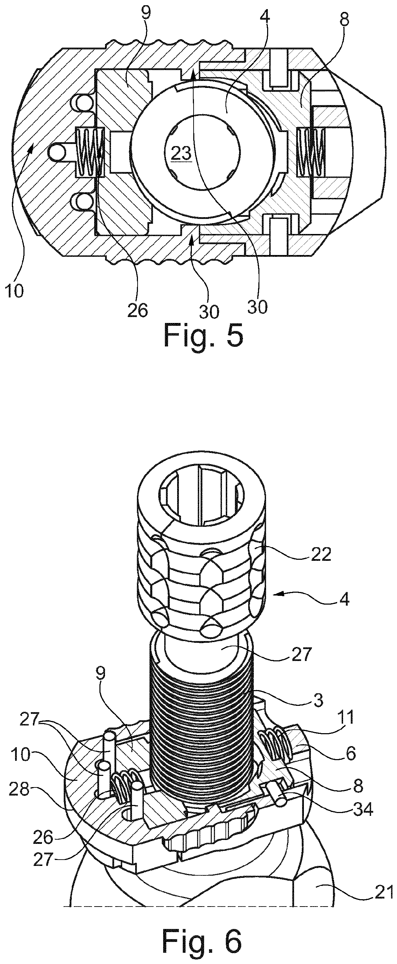

Zum Entriegeln muss der Operateur bei dem beschriebenen Ausführungsbeispiel den Knopf 28 des Entriegelungselements 10 betätigen, um die Gewindestange 4 mit einer reinen Axialbewegung zurückziehen zu können. Der Zustand bei gedrücktem Knopf 28 zum Entriegeln der Gewinde ist in den Figuren 5 und 6 dargestellt. Durch drücken des Knopfs 28 verlagert sich das Betätigungselement 10 radial zur Gewindestange 4 hin. Seine Anschlagzapfen 30 gelangen dabei mit den Anschlagflächen 32 in Anlage, so dass das Innengewindesegment 8 gegen die Radialspannung der Feder 11 nach radial außen fort von der Gewindestange 4 gedrängt wird. Auf diese Weise werden die Gewinde außer Eingriff gebracht. Die Gewindestange 4 kann dann mittels rein axialer Bewegung in die Ausgangsposition, also in die proximale Richtung, zurückgezogen werden.

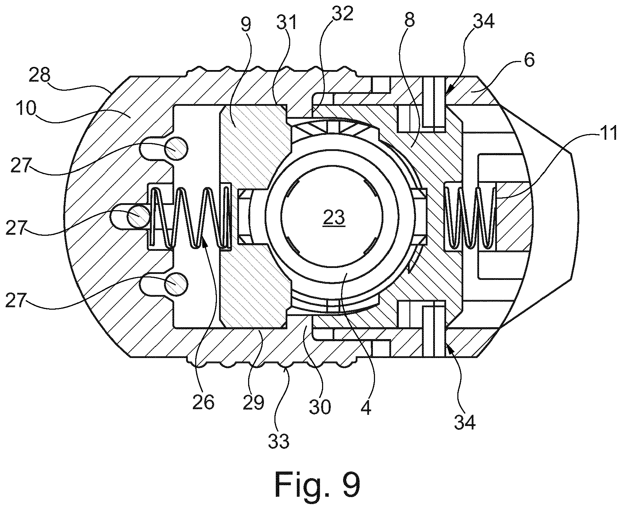

Die Gewindestange 4 kann aufgrund der erfindungsgemäßen Demontagesicherung aber nur soweit zurückgezogen werden, bis das Riegelelement 9 mit dem Anschlag 35 der Gewindestange 4 in Anlage gelangt. Durch die radial nach innen wirkende Vorspannung des Riegelelements 9 mittels der am Betätigungselement abgestützten Feder 26 wird das Riegelelement 9 fortwährend in Richtung der Gewindestange 4 gedrängt. Dabei gleitet es mit seinen Kontaktflächen 38 auf dem Außendurchmesser des Außengewindes 3 ab, bis die Gewindestange in axialer Richtung derart relativplatziert ist, dass das Riegelelement 9 mit dem gewindefreien Abschnitt 36 in Überdeckung gelangt. Durch seine radiale Vorspannung schnappt das Riegelelement 9 radial nach innen (quasi in den gewindefreien Abschnitt hinein) und gelangt bei weiterer Axialbewegung der Gewindestange 4 mit dem Anschlag 35 in Anlage. Die Gewindestange ist dann nicht weiter in proximale Richtung axialbewegbar, so dass das Instrument 1 vor einer unbeabsichtigten vollständigen Demontage der Gewindestange 4 gesichert ist. Die vorgespannte Feder 26 aus dem vorangegangenen Schritt begünstigt das sichere Einschnappen. Selbst wenn der Knopf 28 jetzt losgelassen wird (siehe Figur 9) ist der Mechanismus aufgrund der fortwährenden Vorspannung der Feder 26 so abgestimmt, das das Verriegelungselement 9 weiterhin in den gewindefreien Abschnitt 36 eingreift, mit dem Anschlag 35 der Gewindestange 4 in Anlage ist und ein unbeabsichtigtes Zerlegen verhindert wird. Aus Figur 9 ist auch der Zweck der Stifte 34 zu erkennen: Damit bei losgelassenem Knopf 26 das Innengewindesegment 8 nicht in den gewindefreien Abschnitt 36 eingreift, wird es von den Stiften 34 radial außen zurück gehalten.

Nach dem OP-Ende kann das Instrument 1 trotz der Demontagesicherung zum Reinigen zerlegt werden. Dazu wird das Betätigungselement 10 an den seitlich dafür vorgesehenen geriffelten Griffstrukturen 33 radial nach außen, also von der Gewindestange 4 fort, gezogen. Dies bewirkt, dass die Anschlagzapfen 30 mit den Anschlagflächen 31 des Riegelelements 9 in Anlage kommen und bei fortwährender Radialpositionierung das Riegelelement 9 radial nach außen bewegt wird, bis dieses vom Anschlag 35 der Gewindestange 4 gelöst ist. Die Gewindestange 4 ist dann vollständig freigegeben und kann durch reine Axialbewegung vollständig aus dem Gehäuse 6 entfernt werden.

Da das Außengewinde 3 einen größeren Durchmesser aufweist als der gewindefreie Abschnitt 36, kann das Innengewindesegment 8 nicht in den Hinterschnitt schnappen.

### Bezugszeichenliste

- 1: Instrument
- 2: Kopplungseinheit
- 3: Außengewinde
- 4: Gewindestange
- 5: Rastmechanismus
- 6: Gehäuse
- 7: Durchgangsöffnung
- 8: Innengewindesegment
- 9: Riegelelement
- 10: Entriegelungselement
- 11: zweite Vorspannelement, Druckfeder
- 12: Gewindeflanke
- 13: Gewindeflanke
- 14: Normal zur Längsachse
- 15: Linie
- 16: Gewindeflanke
- 17: Gewindeflanke
- 18: Koppelarme
- 19: Koppelstruktur
- 20: Schraube, Niet
- 21: distales Griffelement
- 22: proximales Griffelement
- 23: Durchgangskanal
- 24: Stabdrücker
- 25: Innengewinde
- 26: erste Vorspannelement Druckfeder
- 27: Führungsstift
- 28: Knopf
- 29: Arm
- 30: Anschlagzapfen
- 31: Anschlagfläche
- 32: Anschlagfläche
- 33: Griffstruktur
- 34: Stift für Innengewindesegment
- 35: Anschlag der Gewindestange
- 36: gewindefreier Abschnitt
- 37: gewindefreier Abschnitt
- 38: Kontaktfläche des Riegelelements für Gewindestange
- 39: Gleitfläche der Riegelelements
- 40: Gleitfläche des Innengewindesegments
- 41: Führung

- α: negativer Flankenwinkel
- β: positiver Flankenwinkel
- γ: Winkel der Anlageflächen

## Patentansprüche

1. Instrument (1) zum Führen eines Stabs in eine Aufnahme eines Implantats, insbesondere einer Pedikelschraube, mit:
einer Kopplungseinheit (2) zum Koppeln des Instruments (1) mit dem Implantat, insbesondere mit einem Kopf der Pedikelschraube,
einer Stabdrückereinheit aufweisend eine mit einem Außengewinde (3) versehene und zur Kopplungseinheit (2) in axialer Richtung positionierbare Gewindestange (4) sowie einen mit dieser drehbar und axialfest gekoppelten Stabdrücker (24); und
zumindest einem zur Kopplungseinheit (2) axialfest angeordneten Innengewindesegment (8), wobei
das Innengewindesegment (8) mit dem Außengewinde (3) der Gewindestange (4) in Eingriff bzw. außer Eingriff bringbar ist,
**dadurch gekennzeichnet, dass**
die Stabdrückereinheit einen Anschlag (35) aufweist, der in radialer Richtung nach außen vorspringt; und
das Instrument (1) ein zur Kopplungseinheit (2) axialfest angeordnetes Riegelelement (9) zum Zusammenwirken mit dem Anschlag (35) aufweist, das gegenüber der Gewindestange (4) in radialer Richtung positionierbar ist und durch ein eine Radialkraft aufbringendes erstes Vorspannelement (26) in Richtung der Gewindestange (4) in einen Eingriff mit dem Anschlag (35) vorgespannt ist.

2. Instrument (1) nach Anspruch 1, **dadurch gekennzeichnet, dass** der Außendurchmesser des Anschlags (35) kleiner ist als der Fußdurchmesser des Außengewindes (3).

3. Instrument (1) nach Anspruch 2, **dadurch gekennzeichnet, dass** in der Gewindestange (4) ein axialer gewindefreier Abschnitt (36) ausgebildet ist, dessen Außendurchmesser kleiner ist als der Fußdurchmesser des Außengewindes (3) und/oder dessen axiale Länge größer ist als die axiale Länge des Innengewindes (25) des Innengewindesegments (8).

4. Instrument (1) nach Anspruch 3, **dadurch gekennzeichnet, dass** der gewindefreie Abschnitt (36) auf der distalen Seite des Außengewindes (3) ausgebildet ist.

5. Instrument (1) nach einem der vorstehenden Ansprüche, **dadurch gekennzeichnet, dass** dieses ein Entriegelungselement (10) aufweist, das mit dem Riegelelement (9) zusammenwirkt, derart, dass das Riegelelement (9) durch eine nutzerseitige Betätigung des Entriegelungselements (10) gegen die durch das erste Vorspannelement (26) aufgebrachte Vorspannung in radialer Richtung nach außen bewegt wird und derart vom Anschlag (35) entkoppelbar ist.

6. Instrument (1) nach Anspruch 5, **dadurch gekennzeichnet, dass** das Entriegelungselement (10) in radialer Richtung relativ zur Gewindestange (4) positionierbar ist.

7. Instrument (1) nach Anspruch 5 oder 6, **dadurch gekennzeichnet, dass** das Entriegelungselement (10) in einem Gehäuse (6) aufgenommen ist und mittels einer Feder (26) in Richtung von der Gewindestange (4) fort vorgespannt ist.

8. Instrument (1) nach einem der Ansprüche 5 bis 7, **dadurch gekennzeichnet, dass** das Riegelelement (9) eine Kontaktfläche (38) für die Gewindestange (4) aufweist, mit der es am Kopfdurchmesser des Außengewindes (3) anliegend in axialer Richtung auf der Gewindestange (4) gleiten kann, ohne mit dem Außengewinde (3) in Gewindeeingriff zu gelangen.

9. Instrument (1) nach einem der vorstehenden Ansprüche, **dadurch gekennzeichnet, dass** Gewindeflanken (12) der Gewindestange (4) und Gewindeflanken (13) des Innengewindesegments (8), die einander zugewandt sind, jeweils einen Hinterschnitt aufweisend ausgebildet sind.

10. Instrument (1) nach einem der vorstehenden Ansprüche, **dadurch gekennzeichnet, dass** das Innengewindesegment (8) gegenüber der Gewindestange (4) in radialer Richtung positionierbar ist und durch ein eine Radialkraft aufbringendes zweites Vorspannelement (11) in Richtung der Gewindestange (4) vorgespannt ist, wobei das Innengewindesegment (8) vorzugsweise in dem Gehäuse (6) aufgenommen ist und vorzugsweise mittels einer Feder (11) als zweites Vorspannelement in Richtung der Gewindestange (4) vorgespannt ist.

11. Instrument (1) nach Anspruch 9 oder 10, **dadurch gekennzeichnet, dass** die mit Hinterschnitt ausgebildeten Flankenwinkel (12) der Gewindestange (4) proximalseitig und die mit einem Hinterschnitt ausgebildeten Flankenwinkel (13) des Innengewindesegments (8) distalseitig angeordnet sind.

12. Instrument (1) nach einem der Ansprüche 6 bis 11, **dadurch gekennzeichnet, dass** das Entriegelungselement (10) und das Innengewindesegment (8) auf einander diametral gegenüberliegenden Seiten der Gewindestange (4) angeordnet sind.

13. Instrument (1) nach einem der vorhergehenden Ansprüche 6 bis 12, **dadurch gekennzeichnet, dass** das Entriegelungselement (10) durch nutzerseitige Betätigung in eine erste Richtung, insbesondere in radialer Richtung auf die Gewindestange (4) zu, sowie in einer zweite Richtung, insbesondere in radialer Richtung von der Gewindestange (4) fort, positionierbar ist.

14. Instrument (1) nach Anspruch 13, **dadurch gekennzeichnet, dass** das Entriegelungselement (10) bei Positionierung in die erste Richtung das Innengewindesegment (8) gegen die Radialkraft des zweiten Vorspannelements (11) von der Gewindestange (4) fortbewegt und mit deren Außengewinde (3) außer Eingriff bringt, und bei Positionierung in die zweite Richtung das Riegelelement (9) gegen die Radialkraft des ersten Vorspannelements (26) von der Gewindestange (4) fort bewegt und mit deren Anschlag (35) außer Eingriff bringt.

15. Instrument (1) nach einem der vorhergehenden Ansprüche, **dadurch gekennzeichnet, dass** die Gewindestange (4) hohl mit einem in axialer Richtung durchgehenden Durchgangskanal (23) ausgebildet ist.

## Claims

1. An instrument (1) for guiding a rod into a receiving area of an implant, in particular a pedicle screw, comprising:
a coupling unit (2) for coupling the instrument (1) to the implant, in particular to a head of the pedicle screw,
a rod pusher unit comprising a threaded rod (4) which is provided with an outer thread (3) and can be positioned in the axial direction relative to the coupling unit (2), and a rod pusher (24) which is coupled to the threaded rod in a rotatable but axially fixed manner; and
at least one inner threaded segment (8) arranged in an axially fixed manner relative to the coupling unit (2), wherein
the inner threaded segment (8) can be brought into engagement or out of engagement with the outer thread (3) of the threaded rod (4),
**characterized in that**
the rod pusher unit has a stop (35) which protrudes outward in the radial direction; and
the instrument (1) has a locking element (9) which is arranged in an axially fixed manner relative to the coupling unit (2) for interacting with the stop (35), wherein the locking element (9) can be positioned in a radial direction relative to the threaded rod (4) and is pretensioned toward the threaded rod (4) so as to engage with the stop (35) by means of a first pretensioning element (26) which applies a radial force.

2. The instrument (1) according to claim 1, **characterized in that** the outer diameter of the stop (35) is smaller than the root diameter of the outer thread (3).

3. The instrument (1) according to claim 2, **characterized in that** an axial unthreaded portion (36) is formed in the threaded rod (4), the outer diameter of which is smaller than the root diameter of the outer thread (3) and/or the axial length of which is larger than the axial length of the inner thread (25) of the inner thread segment (8).

4. The instrument (1) according to claim 3, **characterized in that** the unthreaded portion (36) is formed on the distal side of the outer thread (3).

5. The instrument (1) according to any of the preceding claims, **characterized in that** it has an unlocking element (10) which interacts with the locking element (9) in such a manner that the locking element (9), by actuation of the unlocking element (10) on the part of a user, is moved outwards in the radial direction against the pretension applied by the first pretensioning element (26) and can thus be decoupled from the stop (35).

6. The instrument (1) according to claim 5, **characterized in that** the unlocking element (10) can be positioned in the radial direction relative to the threaded rod (4).

7. The instrument (1) according to claim 5 or 6, **characterized in that** the unlocking element (10) is received in a housing (6) and is pretensioned by means of a spring (26) so as to move away from the threaded rod (4).

8. The instrument (1) according to any of claims 5 to 7, **characterized in that** the locking element (9) has a contact surface (38) for the threaded rod (4) with which it can slide in the axial direction on the threaded rod (4) while resting against the head diameter of the outer thread (3) without coming into thread engagement with the outer thread (3).

9. The instrument (1) according to any of the preceding claims, **characterized in that** thread flanks (12) of the threaded rod (4) and thread flanks (13) of the inner threaded segment (8), which face each other, are each formed so as to have an undercut.

10. The instrument (1) according to any of the preceding claims, **characterized in that** the inner threaded segment (8) can be positioned in the radial direction relative to the threaded rod (4) and is pretensioned toward the threaded rod (4) by a second pretensioning element (11) applying a radial force, the inner threaded segment (8) preferably being received in the housing (6) and pretensioned toward the threaded rod (4) preferably by means of a spring (11) as second pretensioning element.

11. The instrument (1) according to claim 9 or 10, **characterized in that** the flank angles (12) of the threaded rod (4) which are formed with an undercut are arranged on the proximal side and the flank angles (13) of the inner threaded segment (8) which are formed with an undercut are arranged on the distal side.

12. The instrument (1) according to any of claims 6 to 11, **characterized in that** the unlocking element (10) and the inner threaded segment (8) are arranged on diametrically opposite sides of the threaded rod (4).

13. The instrument (1) according to any of the preceding claims 6 to 12, **characterized in that** the unlocking element (10) can be positioned in a first direction by actuation on the part of a user, in particular in the radial direction towards the threaded rod (4), and in a second direction, in particular in the radial direction away from the threaded rod (4).

14. The instrument (1) according to claim 13, **characterized in that**, when positioned in the first direction, the unlocking element (10) moves the inner threaded segment (8) away from the threaded rod (4) against the radial force of the second pretensioning element (11) and brings it out of engagement with its outer thread (3), and, when positioned in the second direction, moves the locking element (9) away from the threaded rod (4) against the radial force of the first pretensioning element (26) and brings it out of engagement with its stop (35).

15. The instrument (1) according to any of the preceding claims, **characterized in that** the threaded rod (4) is hollow with a passage channel (23) continuously extending in the axial direction.

## Revendications

1. Instrument (1) destiné à guider une baguette dans un logement d'un implant, notamment une vis pédiculaire, comprenant :
une unité d'accouplement (2) destinée à accoupler l'instrument (1) avec l'implant, notamment avec une tête de la vis pédiculaire,
une unité de poussoir de baguette présentant une tige filetée (4) pourvue d'un filetage extérieur (3) et pouvant être positionnée dans la direction axiale par rapport à l'unité d'accouplement (2) ainsi qu'un poussoir de baguette (24) accouplé à celle-ci de manière rotative et axialement fixe ; et
au moins un segment de filetage intérieur (8) disposé de manière axialement fixe par rapport à l'unité d'accouplement (2),
le segment de filetage intérieur (8) pouvant être amené en prise ou hors de prise avec le filetage extérieur (3) de la tige filetée (4),
**caractérisé en ce que**
l'unité de poussoir de baguette présente une butée (35) qui fait saillie vers l'extérieur dans la direction radiale ; et
l'instrument (1) présente un élément de verrouillage (9) disposé de manière axialement fixe par rapport à l'unité d'accouplement (2) pour interagir avec la butée (35), lequel élément de verrouillage peut être positionné dans la direction radiale par rapport à la tige filetée (4) et est précontraint en direction de la tige filetée (4) dans une prise avec la butée (35) par un premier élément de précontrainte (26) qui applique une force radiale.

2. Instrument (1) selon la revendication 1, **caractérisé en ce que** le diamètre extérieur de la butée (35) est inférieur au diamètre de base du filetage extérieur (3).

3. Instrument (1) selon la revendication 2, **caractérisé en ce qu'**une section non filetée (36) axiale, dont le diamètre extérieur est inférieur au diamètre de base du filetage extérieur (3) et/ou dont la longueur axiale est supérieure à la longueur axiale du filetage intérieur (25) du segment de filetage intérieur (8), est formée dans la tige filetée (4).

4. Instrument (1) selon la revendication 3, **caractérisé en ce que** la section non filetée (36) est formée sur le côté distal du filetage extérieur (3).

5. Instrument (1) selon l'une des revendications précédentes,
**caractérisé en ce que** celui-ci présente un élément de déverrouillage (10) qui interagit avec l'élément de verrouillage (9) de telle sorte que l'élément de verrouillage (9) est déplacé vers l'extérieur contre la précontrainte appliquée par le premier élément de précontrainte (26) dans la direction radiale par un actionnement côté utilisateur de l'élément de déverrouillage (10) et de façon à pouvoir être désaccouplé de la butée (35).

6. Instrument (1) selon la revendication 5, **caractérisé en ce que** l'élément de déverrouillage (10) peut être positionné dans la direction radiale par rapport à la tige filetée (4).

7. Instrument (1) selon la revendication 5 ou 6, **caractérisé en ce que** l'élément de déverrouillage (10) est logé dans un boîtier (6) et précontraint au moyen d'un ressort (26) dans une direction de manière à s'éloigner de la tige filetée (4).

8. Instrument (1) selon l'une des revendications 5 à 7, **caractérisé en ce que** l'élément de verrouillage (9) présente une surface de contact (38) pour la tige filetée (4), au moyen de laquelle surface de contact il peut glisser dans la direction axiale sur la tige filetée (4) en étant en appui sur le diamètre de tête du filetage extérieur (3), sans entrer en prise de manière filetée avec le filetage extérieur (3).

9. Instrument (1) selon l'une des revendications précédentes,
**caractérisé en ce que** des flancs de filetage (12) de la tige filetée (4) et des flancs de filetage (13) du segment de filetage intérieur (8) qui se font mutuellement face sont formés de manière à présenter respectivement une contre-dépouille.

10. Instrument (1) selon l'une des revendications précédentes,
**caractérisé en ce que** le segment de filetage intérieur (8) peut être positionné dans la direction radiale par rapport à la tige filetée (4) et est précontraint en direction de la tige filetée (4) par un second élément de précontrainte (11) qui applique une force radiale, le segment de filetage intérieur (8) étant de préférence logé dans le boîtier (6) et étant de préférence précontraint en direction de la tige filetée (4) au moyen d'un ressort (11) comme second élément de précontrainte.

11. Instrument (1) selon la revendication 9 ou 10, **caractérisé en ce que** les angles de flancs (12) de la tige filetée (4) formés avec une contre-dépouille sont disposés côté proximal et les angles de flancs (13) du segment de filetage intérieur (8) formés avec une contre-dépouille sont disposés côté distal.

12. Instrument (1) selon l'une des revendications 6 à 11, **caractérisé en ce que** l'élément de déverrouillage (10) et le segment de filetage intérieur (8) sont disposés sur des côtés diamétralement opposés de la tige filetée (4).

13. Instrument (1) selon l'une des revendications 6 à 12,
**caractérisé en ce que** l'élément de déverrouillage (10) peut être positionné par un actionnement côté utilisateur dans une première direction, notamment dans la direction radiale vers la tige filetée (4), ainsi que dans une seconde direction, notamment dans la direction radiale en s'éloignant de la tige filetée (4).

14. Instrument (1) selon la revendication 13, **caractérisé en ce que** l'élément de déverrouillage (10), lors du positionnement dans la première direction, déplace le segment de filetage intérieur (8) à l'écart de la tige filetée (4) contre la force radiale du second élément de précontrainte (11) et l'amène hors de prise avec le filetage extérieur (3) de ladite tige filetée et, lors du positionnement dans la seconde direction, déplace l'élément de verrouillage (9) à l'écart de la tige filetée (4) contre la force radiale du premier élément de précontrainte (26) et l'amène hors de prise avec la butée (35) de ladite tige filetée.

15. Instrument (1) selon l'une des revendications précédentes,
**caractérisé en ce que** la tige filetée (4) est formée creuse avec un canal de passage (23) traversant dans la direction axiale.
